# EUROPEAN PATENT SPECIFICATION

(11) **EP 4 093 486 B1**
(45) Date of publication and mention of the grant of the patent: **08.11.2023**
(21) Application number: 21702056.9
(22) Date of filing: 20.01.2021
(51) Int. Cl.: A61N 1/04, A61N 1/36

(54) **ELECTRODE STRUCTURE**
ELEKTRODENSTRUKTUR
STRUCTURE D'ÉLECTRODE

(30) Priority: 22.01.2020 FI 20205062
(43) Date of publication of application: 30.11.2022
(73) Proprietor: SaluStim Group Oy, 00420 Helsinki (FI)
(72) Inventor: NISKANEN, Tero, 00420 Helsinki (FI); NISSILÄ, Seppo, 00420 Helsinki (FI)
(74) Representative: Kolster Oy Ab
(86) International application number: PCT/FI2021/050028
(87) International publication number: WO 2021/148715

(56) References cited:
- WO-A1-2017/091705
- US-A1- 2016 279 025
- US-A1- 2019 321 636
- US-B2- 9 782 584

## Description

### FIELD OF THE INVENTION

The invention relates to nerve stimulation electrode structures for external ear.

### BACKGROUND

Electrical stimulation is used in muscular and neural training and healing of a human being but it has also wide range of application in prevention and healing of neurological symptoms and disorders of autonomic nervous system without medication and surgical measures. Some examples are: reduction of mental stress and thereby prevention and healing of such stress-associated disorders as anxiety and depression, various pain conditions (migraine, cluster headache, fibromyalgia), memory and cognitive impairments, and sleep disturbances by increasing the parasympathetic activity.

One widely studied and applied method is to stimulate the vagus nerve (VN) either invasively or non-invasively. Non-invasively ones are often named as a transcutaneous vagus nerve stimulation (tVNS or taVNS) as the stimulation is given through the skin with two electrodes.

One interesting place or location for tVNS/taVNS is in an ear in which the vagus nerve branch runs close to upper-posterior corner of the outer ear channel (auditory canal). The vagus nerve branch has been discussed to create nerve connections with the concha and middle outer ear, too.

Electrical stimulation is widely using skin electrodes similar to ECG or EMG monitoring purposes. A typical electrode is flat with 30 mm in dimeter. It has a sticker on the outer side for attachment the electrode to a skin, a metal or other conductive material in the middle. The contact may be enhanced with a gel on the top of the conductive element to ensure a good electrical contact to the skin. Two electrodes are needed. These are typically attached on the both side of stimulation body part or side by side close to the stimulation area. The electrodes are connected electrically by wires to the stimulation device which produces stimulation pulses in current and voltage through two outputs to be connected to the two electrodes. In stimulation, the stimulation voltage or current is driven between the electrodes to generate a stimulation current in the tissue under the skin and between the electrodes. The known flat electrodes cannot be used inside the ear channel as it is not possible to use stickers inside the ear channel.

Regarding stimulation of vagus nerve via ear and especially stimulation of aurical branch of the vagus nerve via ear, the known electrode structures are included in ear plugs or ear phones, in some cases the ear plugs are part of a headset having ear plugs combined by a headband. Some examples are shown in US4966164, US20050165460 and US9782584. The known electrode structures are complicated to manufacture and they are expensive. The known structures are also difficult connect to the stimulation device and challenging to be changed easily. Due to a skin contact part there is a need to change and replace electrode parts, so there is a real need to solve the above mentioned problems. Ears and ear channels are different regarding size and therefore there is a is need for an electrode structure which can be modified or manufactured easily and can be fitted to ear in a suitable manner.

### BRIEF DESCRIPTION

An object of the present invention to provide an electrode structure so as to solve or alleviate the above mentioned problems. The object of the invention is achieved by an electrode structure which is characterized by what is stated in the independent claim. The preferred embodiments of the invention are disclosed in the dependent claims. Aspects, embodiments and examples disclosed herein which do not fall within the scope of the appended claims do not form part of the invention, and are merely provided for illustrative purposes.

The invention is based on the idea of bendability of the electrode structure so that when inclined the electrode structure can be inputted to ear channel.

An advantage of the new electrode structure is that structure enables good contact with the ear channel and especially with the outer wall of the ear channel. New electrode structure is easy and cost effective to be manufactured.

### BRIEF DESCRIPTION OF THE DRAWINGS

In the following the invention will be described in greater detail by means of preferred embodiments with reference to the accompanying drawings, in which:
Figure 1 shows a front side view of a symmetrical electrode structure, shown without snap connectors
Figure 2 shows a front side view of a symmetrical electrode structure,
Figure 3 shows a back side view of a symmetrical electrode structure
Figure 4 shows the schematic cross-section of the multi-layered electrode structure, shown with the stimulation device related counterparts of the snap connectors of the electrode structure,
Figure 5 shows a front side view of a non-symmetrical electrode structure,
Figure 6 shows the non-symmetrical electrode structure in inclined position,
Figure 7 shows the front side of the electrode structure, said electrode structure having air channel through the bending area,
Figure 8 is a schematic top view of a head of a person,
Figure 9 is a schematic top view of a head of a person, shown with electrode structure and related stimulation device,
Figure 10 shows how the electrode structure is connected to the stimulation device.

### DETAILED DESCRIPTION OF THE INVENTION

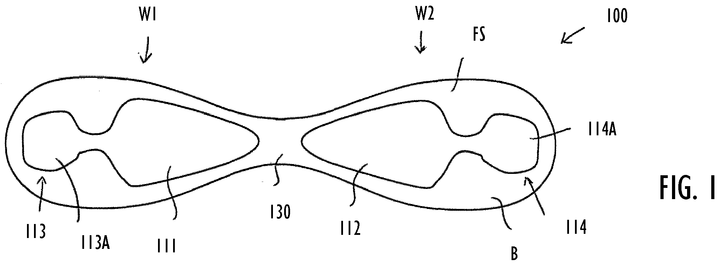

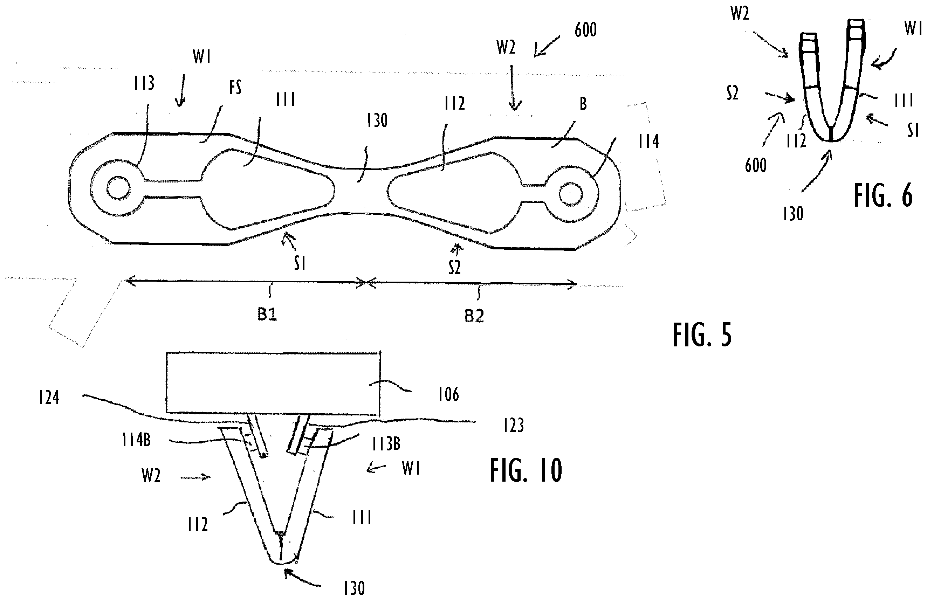

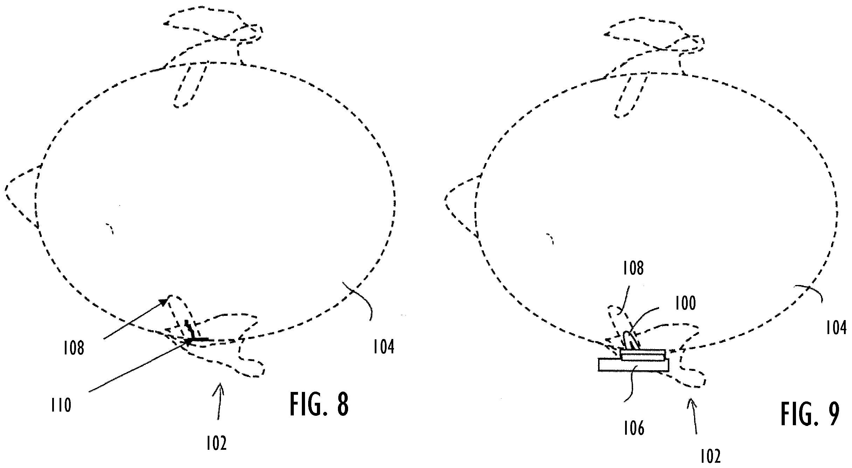

Relating to figures 1-3 and 8-9, there is shown a nerve stimulation electrode structure 100 for external ear 102 on the head 104 of the person. Electrode structure 100 is connected to a stimulation device 106. In inclined position (figures 6 and 9) the electrode structure 100, 600 is adapted to be inputted to the ear channel 108 (auditory canal) of the person. End of the nerve 110 such as vagus nerve 110 is shown in figure 8, it represents ABVN which is auricular branch of vagus nerve.

The electrode structure 100 comprises a first electrode 111, a second electrode 112 and a base B for supporting the electrodes. Electrodes 111, 112 are electrically conductive. Regarding electrodes 111, 112, electrode can be for example a carbon-paste electrode (CPE) made from a mixture of conducting graphite powder and a pasting liquid. The conductive material can be also silver, gold, conductive plastic or organic material which is connected to an electrode base material using a gel, paste, printing or similar method.

Additionally, the electrode structure 100 further comprises connecting points 113, 114 for the electrodes 111, 112 for creating connection to a stimulation device 106. In an embodiment, connecting points 113, 114 comprises areas such as end areas 113A, 114A and connectors 113B, 114B such as snap connectors 113B, 114B having galvanic contact with the connecting areas 113A, 114A of the related electrodes. Connecting area 113A is in galvanic contact with the related first electrode 111 and the connecting area 114A is in galvanic contact with the related second electrode 112. Connector 113B such as snap connector is in galvanic contact with the related connecting area 113A of the first electrode 111, and likewise, connector 114B such as snap connector is in galvanic contact with the related connecting area 114A of the second electrode 112. Also the connection points 113, 114 and parts (113A, 113B, 114A, 114B) thereof are supported on the base B.

The galvanic contact between electrode 111 and connecting area 113A is preferable made using the same material and manufacturing method as for electrode 111, therefore the electrode 111 and connecting area 113A actually belong to the same conductive layer, the same is true also regarding relation of second electrode 112 and connecting area 114A.

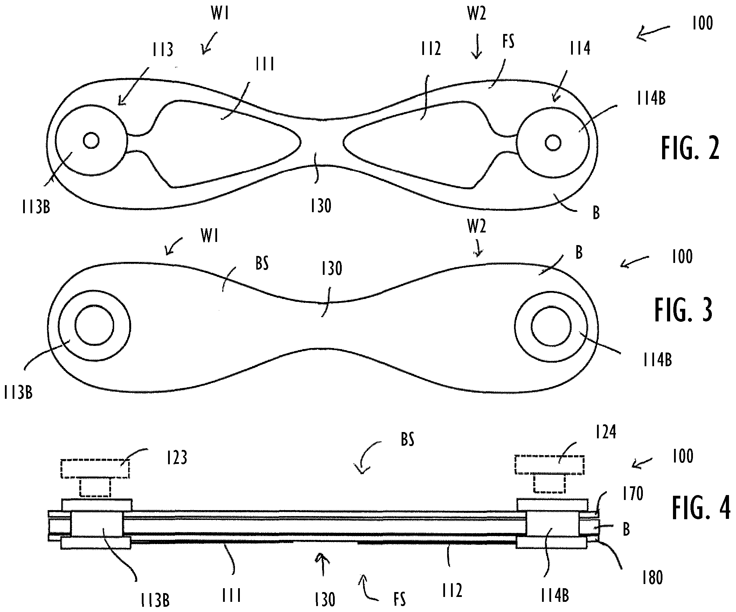

Snap connectors 113B, 114B create electrical (galvanic)contact through the structure as can be seen in figure 4 and thereby they are in contact to respective conductive electrodes 111, 112, i.e electrode layers 111, 112. Figure 2 is a frontside view of the electrode structure 100, therefore showing the front side FS i.e side where the electrodes 111, 112 are, and therefore figure 2 shows the back side of the snap connectors 113B, 114B. Figure 3 is a back-side view of the electrode structure 100, and therefore figure 3 shows the front side of the snap connectors 113B, 114B. Front side of the snap connectors 113B, 114B are adapted to be connected to corresponding counterparts 123, 124 at the stimulation device 106.

In figure 4, the electrode structure 100 is not bended/inclined yet, therefore the location of the snap connectors 113B 114B at the electrode structure 100 in relation to location of the counterparts 123, 124 at the stimulation device is not as shown in figure 4. Therefore, figure 10 shows how the bended/inclined V-shaped electrode structure 100 is connected to the stimulation device 106. Snap connector 113B at the electrode structure 100 meets the counterpart 123 at the stimulation device 106, and similarly the snap connector 114B at the electrode structure 100 meets the counterpart 124 at the stimulation device 106.

The connecting areas 113A, 114A do not need to be remote from the actual electrodes 111, 112, therefore the connecting areas can also be formed from egde areas of the electrodes. The galvanic contact between a snap connector 113B, 114B and respective connecting area 113A, 114A is preferable made as a mechanical pressing force. Snap connectors 113B, 114B are made of parts, which are pressed together when the connecting area 113A, 114A is placed to be pressed between snap connector parts to form a fixed snap connector structure. See Fig 4, 113B parts pressed together and connecting area (113A shown in fig. 1) being between those parts of the snap connector 113B.

In an embodiment, female part of the snap connector 113B (114B) is on the electrode structure 100 (female part opening itself at the back side BS of the electrode structure) and male part of the snap connector 113B (114B) is on the stimulation device 106. This makes it easier to connect the electrode structure 100 and stimulation device 106 together.

Stimulation device 106, whereto the electrode structure 100 is connected, comprises counterparts 123, 124 for the connecting points 113, 114, in practise counterparts 123, 124 are counterparts for the snap connectors 113B, 114B. A stimulation device is producing electrical stimulation pulse to electrodes 111, 112. One simulation pulse can be typically 10-80 V peak to peak, (for example being first 40V positive and them 40V negative. a typical pulse length is 20 -500 microseconds. Typically, the stimulation sequence consists of a series of pulses for example 5-100 pulses. In other way the pulse repetition rate can 5-30 Hz. The sequences can be repeated for example every hour or three times per day depending on the treatment or healing procedure. The stimulation device 106 can control the amplitude, voltage and current depending on the skin contact impedance. The stimulation device 106 may also drive a constant current pulses varying voltage of the pulse as a function of the skin impedance. The stimulation 106 device may also produce audio output for enhancing stimulation, guiding breathing or providing voice commands to a user. The stimulation device is a battery powered and possible rechargeable. The stimulation device may have input control buttons for setting other device on and off, adjusting audio volume and adjusting stimulation amplitude (voltage or current).

Regarding the important feature on the electrode structure 100, at least in the area between the electrodes 111, 112, the base B comprises a flexible bending area 130 for enabling the electrode structure 100 to be bent to an inclined position forming a first wing W1 for the first electrode 111 and a second wing W2 for the second electrode 112 for enabling electrode-contact to the ear (ear channel 108) on both outer sides S1, S2 of the bended electrode structure 100 as shown in figure 9. Bended/inclined electrode structure can be seen in figure 9 (electrode structure 100) and in figure 6 (electrode structure 600), and in figure 10. The front side FS of the electrode structure 100 has both outer sides S1, S2, when the electrode structure seen in the bended/inclined position (V-form). Outer side S1 of the electrode structure 100 is at the wing W1, and the outer side S2 of the electrode structure 100 is at the wing W2. The front side FS of the electrode structure 100 is the side where the electrodes 111, 112 are, whereas the back side BS of electrode structure 100 is the opposite side of the electrode structure 100.

The base B is a planar structure, enabling the electrode structure 100 to have very limited thickness also when in inclined position. Figure 6 shows electrode structure 600 in inclined position. Figure 9 shows electrode structure 100 partially inside the ear channel 108 and connected to stimulation device 106.

Planar structure of the base B also provides enough space for the electrodes 111, 112 and helps the bendability of the electrode structure 100 to be suitable and controlled. The planar base B of the electrode structure 100 is planar at least on that side of the base where the electrodes 111, 112 are.

In an embodiment, substantially the whole base B is formed from bendable material. In a further embodiment, base is made from polyethylene. One suitable example or base B is Medical Foam Tape 1773, made by 3M^{™}.

In an embodiment, the connecting points (having connectors 113B, 114B) are adapted to be mechanically connected to counterparts 123, 124 at the stimulation device 106 so as to lock the wings W1, W2 of the electrode structure 100 (in fig. 9), 600 (600 in fig 6) to a fixed inclined position compared to each other.

The electrode structure 100 has longitudinal length and transversal width. Electrodes 111, 112 are consecutive (one after another) to each other, when the electrode structure 100 is seen in longitudinal direction.

In an embodiment, at the bending area 130 the transversal width of the base B is less than the transversal width of the base at the wings of the electrode structure. For example, the transversal width of the base B at the bending area 130 is 2-6mm such as 4mm, whereas elsewhere the full width of the base B is 6-12mm such as 10mm. As an example, the longitudinal length the base B (same as the longitudinal length of the electrode structure 100) is 20-60mm such as 50mm.

Referring especially to figure 4, in an embodiment, the electrode structure 100 is a multi-layered structure. Then, the multi-layered electrode structure 100 includes base layer B (the base) and electrode layers 111, 112 (the electrodes 111, 112). Additionally, the multi-layered electrode structure 100 may have stiffener layer 170 and/or middle layer 180. Middle layer 180 is between the base layer and electrode layers 111, 112, said middle layer acting as printing surface of the printed electrode layers 111, 112. Base B i.e the base layer B may have several sub-layers, for example three sub-layers that are glued together. The stiffener layer 170 may be on top or bottom of the foam-made base layer B, or between the sub-layers of the base layer.

As mentioned earlier, the connecting points 113, 114 comprise snap connectors 113B, 114B. The role of the snap connectors 113B, 114B is to provide galvanic contact from the electrodes 111, 112 (via connecting areas 113A, 114A) to the counterparts at the stimulation device 106. Additionally, the role of the snap connectors 113B, 114B is to provide mechanical connection/support between the electrode structure 100 and the stimulation device 106.

In an embodiment, the location of the bending area 130 is at the center regarding the longitudinal length of the electrode structure 100. This is true especially with the symmetrical version of the electrode structure, shown in figures 1-4.

Figures 1-4 relate to symmetrical version of the electrode structure 100. In this embodiment, electrode structure 100 is a symmetrical structure wherein both connecting points 113, 114 have substantially similar distance to the bending area (centre area) 130.

In a different embodiment, as shown in figures 5 and 6, the electrode structure 600 is a non-symmetrical structure wherein the connecting points 113A, 114 have different distance to the bending area 130 (which is not exactly at the center). In figure 6, distance B1 is distance from connecting point 113 (snap connector 113B) to bending area 130, and distance B2 is distance from connecting point (snap connector 114B) to bending area 130. In this non-symmetrical electrode structure 600, distance B1is greater than distance B2.

Electrode structure 100 is removably attached to the stimulation device 106. Connectors such as snap connectors 113B, 114B and their counterparts 123, 124 make it possible that the electrode structure 100 is easily attached to or detached from the stimulation device 106.

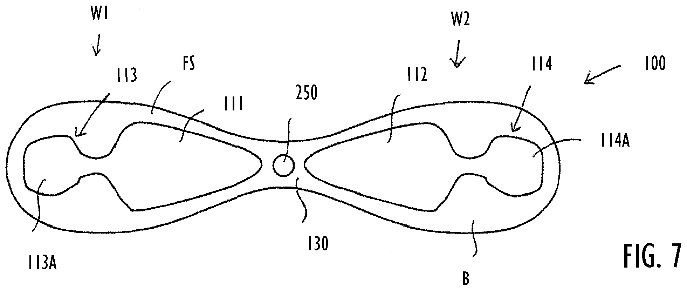

Figure 7 shows the front side FS of the electrode structure 700, said electrode structure having air channel 250 through the bending area 130. This is beneficial, because it makes the person/patient able to keep his/her ability to hear, for example to hear instructions from a doctor or a nurse. It also makes possible to give audio output from the stimulation device if the device is equipped with a speaker or piezo and audio output properties. The audio output can be breathing guidance sound or voice commands and instruction.

Air channel 250 is a hole extending through the electrode structure 100, said channel i.e hole extending between front side FS and back side BS of the electrode structure 100.

Regarding other features than symmetry/non-symmetry features, the above mentioned features and details relating to figures 1- 4, 7-10 are true also for the non-symmetrical version shown in figures 5-6.

It will be obvious to a person skilled in the art that, as the technology advances, the inventive concept can be implemented in various ways. The invention and its embodiments are not limited to the examples described above but may vary within the scope of the claims.

## Claims

1. Nerve stimulation electrode structure for external ear, said electrode structure (100) comprising a first electrode (111), a second electrode (112), a base (B) for supporting the electrodes, said electrode structure further comprising connecting points (113, 113A, 113B, 114, 114A, 114B) for the electrodes for creating connection to a stimulation device (106), wherein at least in the area between the electrodes (111, 112) the base (B), said base (B) being planar, comprises a flexible bending area (130) for enabling the electrode structure (100) to be bent to an inclined position forming a first wing (W1) for the first electrode and a second wing (W2) for the second electrode for enabling electrode-contact to the ear on both outer sides of the bended electrode structure.

2. The electrode structure as claimed in claim 1, **characterized in that** substantially the whole base (B) is formed from bendable material

3. The electrode structure as claimed in claim 1 or 2, **character**- **ized** in that base (B) is made from polyethylene.

4. The electrode structure as claimed in any of claims 1-3, **charac**- **terized** in that the connecting points (113, 113B, 114, 114B) are adapted to be mechanically connected to counterparts (123, 124) at said stimulation device (106) so as to lock the wings (W1, W2) of the electrode structure to a fixed inclined position compared to each other.

5. The electrode structure as claimed in any of claims 1-4, **charac**- **terized** in that the electrode structure (100) has longitudinal length and transversal width, and that at the bending area (130) the transversal width of the base (B) is less than the transversal width of the base (B) at the wings (W1, W2) of the electrode structure (100).

6. The electrode structure as claimed in any of claims 1-5, **charac**- **terized** in that the electrode structure (100) is a multi-layered structure.

7. The electrode structure as claimed in claim 5, **characterized in that** the multi-layered electrode structure includes base layer (B) and electrode layers (111, 112) and at least one of: stiffener layer (170), middle layer (180) between the base layer (B) and electrode layers (111, 112), said middle layer (170) acting as printing surface of the printed electrode layers (111, 112).

8. The electrode structure as claimed in any of claims 1-7, **charac**- **terized** in that the connecting points (113, 114) comprise snap connectors (113B, 114B).

9. The electrode structure as claimed in any of claims 1-8, **charac**- **terized** in that the planar base (B) of the electrode structure is planar at least on that side of the base (B) where the electrodes (111, 112) are.

10. The electrode structure as claimed in any of claims 1-9, **charac**- **terized** in that the electrode structure (100) is a symmetrical structure wherein both connecting points (113, 114) have substantially similar distance to the bending area (130).

11. The electrode structure as claimed in any of claims 1-9, **charac**- **terized** in that the electrode structure (100) is a non-symmetrical structure wherein the connecting points (113, 114) have different distance to the bending area (130).

12. The electrode structure as claimed in any of claims 1-10, **characterized in that** the electrode structure (100) has longitudinal length and transversal width, and wherein the location of the bending area (130) is at the center regarding the longitudinal length of the electrode structure (100).

## Patentansprüche

1. Nervenstimulationselektrodenstruktur für ein Außenohr, wobei die Elektrodenstruktur (100) eine erste Elektrode (111), eine zweite Elektrode (112), eine Basis (B) zum Stützen der Elektroden umfasst, wobei die Elektrodenstruktur zum Herstellen einer Verbindung mit einer Stimulationsvorrichtung (106) ferner Verbindungspunkte (113, 113A, 113B, 114, 114A, 114B) für die Elektroden umfasst, wobei die Basis (B) - wobei die Basis (B) planar ist - zum Ermöglichen des Biegens der Elektrodenstruktur (100) in eine geneigte Position mindestens in dem Bereich zwischen den Elektroden (111, 112) einen flexiblen Biegebereich (130) umfasst, wodurch zum Ermöglichen eines Elektrodenkontakts mit dem Ohr auf beiden Außenseiten der gebogenen Elektrodenstruktur ein erster Flügel (W1) für die erste Elektrode und ein zweiter Flügel (W2) für die zweite Elektrode gebildet wird.

2. Elektrodenstruktur nach Anspruch 1, **dadurch gekennzeichnet ist, dass** im Wesentlichen die gesamte Basis (B) aus einem biegbaren Material gebildet ist

3. Elektrodenstruktur nach Anspruch 1 oder 2, **dadurch gekennzeichnet ist, dass** die Basis (B) aus Polyethylen besteht.

4. Elektronenstruktur nach einem der Ansprüche 1-3, **dadurch gekennzeichnet, dass** die Verbindungspunkte (113, 113B, 114, 114B) angepasst sind, an der Stimulationsvorrichtung (106) mit Gegenstücken (123, 124) mechanisch verbunden zu sein, um die Flügel (W1, W2) der Elektrodenstruktur in einer festen geneigten Position zueinander zu verriegeln.

5. Elektrodenstruktur nach einem der Ansprüche 1-4, **dadurch gekennzeichnet, dass** die Elektrodenstruktur (100) eine Längslänge und eine Querbreite aufweist und dass der Biegebereich (130) der Querbreite der Basis (B) kleiner ist als die Querbreite der Basis (B) an den Flügeln (W1, W2) der Elektrodenstruktur (100).

6. Elektrodenstruktur nach einem der Ansprüche 1-5, **dadurch gekennzeichnet, dass** die Elektrodenstruktur (100) eine mehrschichtige Struktur ist.

7. Elektrodenstruktur nach Anspruch 5, **dadurch gekennzeichnet, dass** die mehrschichtige Elektrodenstruktur eine Basisschicht (B) und Elektrodenschichten (111, 112) und mindestens eines von Folgendem beinhaltet: eine Versteifungsschicht (170), eine Mittelschicht (180) zwischen der Basisschicht (B) und den Elektrodenschichten (111, 112), wobei die Mittelschicht (170) als eine Druckfläche der gedruckten Elektrodenschichten (111, 112) fungiert.

8. Elektrodenstruktur nach einem der Ansprüche 1-7, **dadurch gekennzeichnet, dass** die Verbindungspunkte (113, 114) Rastverbinder (113B, 114B) umfassen.

9. Elektrodenstruktur nach einem der Ansprüche 1-8, **dadurch gekennzeichnet, dass** die planare Basis (B) der Elektrodenstruktur mindestens auf der Seite der Basis (B), auf der sich die Elektroden (111, 112) befinden, planar ist.

10. Elektrodenstruktur nach einem der Ansprüche 1-9, **dadurch gekennzeichnet, dass** die Elektrodenstruktur (100) eine symmetrische Struktur ist, wobei beide Verbindungspunkte (113, 114) einen im Wesentlichen ähnlichen Abstand zum Biegebereich (130) aufweisen.

11. Elektrodenstruktur nach einem der Ansprüche 1-9, **dadurch gekennzeichnet, dass** die Elektrodenstruktur (100) eine nicht symmetrische Struktur ist, wobei die Verbindungspunkte (113, 114) einen unterschiedlichen Abstand zum Biegebereich (130) aufweisen.

12. Elektrodenstruktur nach einem der Ansprüche 1-10, **dadurch gekennzeichnet, dass** die Elektrodenstruktur (100) eine Längslänge und eine Querbreite aufweist, und wobei der Ort des Biegebereichs (130) mit Bezug auf die Längslänge der Elektronenstruktur (100) in der Mitte liegt.

## Revendications

1. Structure d'électrode de stimulation nerveuse pour l'oreille externe, ladite structure d'électrode (100) comprenant une première électrode (111), une deuxième électrode (112), une base (B) pour supporter les électrodes, ladite structure d'électrode comprenant en outre des points de connexion (113, 113A, 113B, 114, 114A, 114B) pour les électrodes afin de créer une connexion à un dispositif de stimulation (106), dans laquelle
au moins dans la zone se trouvant entre les électrodes (111, 112), la base (B) qui est plane comprend une zone de pliage flexible (130) permettant de plier la structure d'électrode (100) en position inclinée, formant une première aile (W1) pour la première électrode et une deuxième aile (W2) pour la deuxième électrode, afin de permettre un contact d'électrode avec l'oreille sur les deux côtés extérieurs de la structure d'électrode pliée.

2. Structure d'électrode selon la revendication 1, **caractérisée en ce que** la quasi-totalité de la base (B) est formée d'un matériau pliable.

3. Structure d'électrode selon la revendication 1 ou 2, **caractérisée en ce que** la base (B) est réalisée en polyéthylène.

4. Structure d'électrode selon l'une quelconque des revendications 1 à 3, **caractérisée en ce que** les points de connexion (113, 113B, 114, 114B) sont adaptés pour être connectés mécaniquement à leurs équivalents (123, 124) sur ledit dispositif de stimulation (106) de manière à bloquer les ailes (W1, W2) de la structure d'électrode dans une position inclinée fixe l'une par rapport à l'autre.

5. Structure d'électrode selon l'une quelconque des revendications 1 à 4, **caractérisée en ce que** la structure d'électrode (100) a une longueur longitudinale et une largeur transversale, et **en ce qu'**au niveau de la zone de pliage (130), la largeur transversale de la base (B) est inférieure à la largeur transversale de la base (B) au niveau des ailes (W1, W2) de la structure d'électrode (100).

6. Structure d'électrode selon l'une quelconque des revendications 1 à 5, **caractérisée en ce que** la structure d'électrode (100) est une structure multicouche.

7. Structure d'électrode selon la revendication 5, **caractérisée en ce que** la structure multicouche d'électrode comporte une couche de base (B) et des couches d'électrode (111, 112), et au moins une pami : une couche de raidissement (170), une couche intermédiaire (180) entre la couche de base (B) et les couches d'électrode (111, 112), ladite couche intermédiaire (170) agissant comme surface d'impression des couches d'électrode (111, 112) imprimées.

8. Structure d'électrode selon l'une quelconque des revendications 1 à 7, **caractérisée en ce que** les points de connexion (113, 114) comprennent des connecteurs encliquetables (113B, 114B).

9. Structure d'électrode selon l'une quelconque des revendications 1 à 8, **caractérisée en ce que** la base (B) plane de la structure d'électrode est plane au moins sur le côté de la base (B) où se trouvent les électrodes (111, 112).

10. Structure d'électrode selon l'une quelconque des revendications 1 à 9, **caractérisée en ce que** la structure d'électrode (100) est une structure symétrique dans laquelle les deux points de connexion (113, 114) sont à une distance sensiblement similaire de la zone de pliage (130).

11. Structure d'électrode selon l'une quelconque des revendications 1 à 9, **caractérisée en ce que** la structure d'électrode (100) est une structure non symétrique dans laquelle les points de connexion (113, 114) sont à des distances différentes de la zone de pliage (130).

12. Structure d'électrode selon l'une quelconque des revendications 1 à 10, **caractérisée en ce que** la structure d'électrode (100) a une longueur longitudinale et une largeur transversale, et dans laquelle l'emplacement de la zone de pliage (130) est au centre de la longueur longitudinale de la structure d'électrode (100).
